# EUROPEAN PATENT APPLICATION

(11) **EP 3 078 741 A1**
(43) Date of publication of application: **12.10.2016**
(21) Application number: 14868187.7
(22) Date of filing: 03.12.2014
(51) Int. Cl.: C12N 9/16, A21D 2/34, A21D 13/00, A23G 3/00, A23G 3/34, A23L 27/60, A23L 15/00, C12N 15/09

(54) **FLAVOR-IMPROVING ENZYME COMPOSITION, METHOD FOR SUPPRESSING OCCURRENCE OF UNPLEASANT ODOR, AND METHOD FOR MANUFACTURING FOOD WITH REDUCED UNPLEASANT ODOR**

(30) Priority: 05.12.2013 JP 2013252510
(71) Applicant: Nagase ChemteX Corporation, Osaka-shi, Osaka 550-8668 (JP)
(72) Inventor: SHIRASAKA, Naoki, Fukuchiyama-shi Kyoto 620-0853 (JP); ATSUMI, Yuta, Fukuchiyama-shi Kyoto 620-0853 (JP)
(74) Representative: Schlich, George
(86) International application number: PCT/JP2014/082050
(87) International publication number: WO 2015/083757

(57) **Abstract**

A flavor-improving enzyme composition for reducing an unpleasant odor in a food or beverage is disclosed, the composition containing an enzyme exhibiting phospholipase A2 activity with lipase activity/phospholipase A2 activity of not more than 0.005, as an active ingredient.

SEQUENCE LISTING

<110>  Nagase ChemteX Corporation

<120>  Enzyme composition for improving flavor, method for controling
       odor incidence, and odor incidence-controlled food

<130>  P2-14N13106

<150>  JP2013-252510
<151>  2013-12-05

<160>  6

<170>  PatentIn version 3.5

<210>  1
<211>  456
<212>  DNA
<213>  Streptomyces violaceoruber


<220>
<221>  CDS
<222>  (1)..(456)
<223>  Phospholipase A2

<400>  1
gtg cgc acc acc acc agg acc cgg acc acg ctc gcc gcc gtc ggt gcg        48
Met Arg Thr Thr Thr Arg Thr Arg Thr Thr Leu Ala Ala Val Gly Ala
1               5                   10                  15

gcg ctc gcc ctc ggc gtc gcc gcc gcg ccc gcc cag gcg gcc ccc gcg        96
Ala Leu Ala Leu Gly Val Ala Ala Ala Pro Ala Gln Ala Ala Pro Ala
            20                  25                  30

gac aag ccc cag gta ctc gcc tcc ttc acg cag acc agc gcg tcc agc       144
Asp Lys Pro Gln Val Leu Ala Ser Phe Thr Gln Thr Ser Ala Ser Ser
        35                  40                  45

cag aac gcc tgg ctc gcg gcc aac cgg aac cag tcc gcc tgg gcc gcc       192
Gln Asn Ala Trp Leu Ala Ala Asn Arg Asn Gln Ser Ala Trp Ala Ala
    50                  55                  60

tac gag ttc gac tgg tcc acg gac ctg tgc acc cag gcg ccc gac aac       240
Tyr Glu Phe Asp Trp Ser Thr Asp Leu Cys Thr Gln Ala Pro Asp Asn
65                  70                  75                  80

ccc ttc ggc ttc ccg ttc aac acg gcc tgc gcg cgc cac gac ttc ggt       288
Pro Phe Gly Phe Pro Phe Asn Thr Ala Cys Ala Arg His Asp Phe Gly
                85                  90                  95

tac cgc aac tac aag gcg gcg ggc agc ttc gac gcc aac aag agc cgt       336
Tyr Arg Asn Tyr Lys Ala Ala Gly Ser Phe Asp Ala Asn Lys Ser Arg
            100                 105                 110

atc gac agc gcc ttc tac gag gac atg aag cgc gtc tgc acc ggc tac       384
Ile Asp Ser Ala Phe Tyr Glu Asp Met Lys Arg Val Cys Thr Gly Tyr
        115                 120                 125

acc ggc gag aag aac acg gcc tgc aac agc acc gcc tgg acc tac tac       432
Thr Gly Glu Lys Asn Thr Ala Cys Asn Ser Thr Ala Trp Thr Tyr Tyr
    130                 135                 140

cag gcc gtc aag atc ttc ggc tga                                    456
Gln Ala Val Lys Ile Phe Gly
145                 150

<210>  2
<211>  151
<212>  PRT
<213>  Streptomyces violaceoruber

<400>  2

Met Arg Thr Thr Thr Arg Thr Arg Thr Thr Leu Ala Ala Val Gly Ala
1               5                   10                  15

Ala Leu Ala Leu Gly Val Ala Ala Ala Pro Ala Gln Ala Ala Pro Ala
            20                  25                  30

Asp Lys Pro Gln Val Leu Ala Ser Phe Thr Gln Thr Ser Ala Ser Ser
        35                  40                  45

Gln Asn Ala Trp Leu Ala Ala Asn Arg Asn Gln Ser Ala Trp Ala Ala
    50                  55                  60

Tyr Glu Phe Asp Trp Ser Thr Asp Leu Cys Thr Gln Ala Pro Asp Asn
65                  70                  75                  80

Pro Phe Gly Phe Pro Phe Asn Thr Ala Cys Ala Arg His Asp Phe Gly
                85                  90                  95

Tyr Arg Asn Tyr Lys Ala Ala Gly Ser Phe Asp Ala Asn Lys Ser Arg
            100                 105                 110

Ile Asp Ser Ala Phe Tyr Glu Asp Met Lys Arg Val Cys Thr Gly Tyr
        115                 120                 125

Thr Gly Glu Lys Asn Thr Ala Cys Asn Ser Thr Ala Trp Thr Tyr Tyr
    130                 135                 140

Gln Ala Val Lys Ile Phe Gly
145                 150

<210>  3
<211>  453
<212>  DNA
<213>  Streptomyces avermitilis

<220>
<221>  CDS
<222>  (1)..(453)
<223>  Phospholipase A2

<400>  3
atg cgc cgt cgc gtc gcc gcc acg ctg gcc acc acc gcc ctc gcg ctg          48
Met Arg Arg Arg Val Ala Ala Thr Leu Ala Thr Thr Ala Leu Ala Leu
1               5                   10                  15

acc gcc gcc ctc gta ccc gtc acg tcg gca tcc gcc gca ccc gcc gac          96
Thr Ala Ala Leu Val Pro Val Thr Ser Ala Ser Ala Ala Pro Ala Asp
            20                  25                  30

aag gcg acg gtc ctc agc aac tgg acc cag acc agc gcc gcc agc tac         144
Lys Ala Thr Val Leu Ser Asn Trp Thr Gln Thr Ser Ala Ala Ser Tyr
        35                  40                  45

aac gcc ttc ttc gcg gcc cgc gcc gac cag ggc gcc tgg agc gcg tac         192
Asn Ala Phe Phe Ala Ala Arg Ala Asp Gln Gly Ala Trp Ser Ala Tyr
    50                  55                  60

ggc ttc gac tgg tcc acc gac tac tgc acc acc tcg ccg gac aac ccg         240
Gly Phe Asp Trp Ser Thr Asp Tyr Cys Thr Thr Ser Pro Asp Asn Pro
65                  70                  75                  80

ttc ggc ttc ccc ttc gcc aac tcg tgc gtc cgg cac gac ttc ggc tac         288
Phe Gly Phe Pro Phe Ala Asn Ser Cys Val Arg His Asp Phe Gly Tyr
                85                  90                  95

cgc aac tac acg gcg gcc ggc tcc ttc gac gcc aac aag tcc cgc ctg         336
Arg Asn Tyr Thr Ala Ala Gly Ser Phe Asp Ala Asn Lys Ser Arg Leu
            100                 105                 110

gac agc gcc ttc tac gcc gac ctc aag cgg gtc tgc acg gcc tac tcc         384
Asp Ser Ala Phe Tyr Ala Asp Leu Lys Arg Val Cys Thr Ala Tyr Ser
        115                 120                 125

gga gtg aag aag gcg tcg tgc gac gcg acg gcc tgg acc tac tac cag         432
Gly Val Lys Lys Ala Ser Cys Asp Ala Thr Ala Trp Thr Tyr Tyr Gln
    130                 135                 140

gcg gtc gtc aac ctg ggc tga                                             453
Ala Val Val Asn Leu Gly
145                 150


<210>  4
<211>  150
<212>  PRT
<213>  Streptomyces avermitilis

<400>  4

Met Arg Arg Arg Val Ala Ala Thr Leu Ala Thr Thr Ala Leu Ala Leu
1               5                   10                  15


Thr Ala Ala Leu Val Pro Val Thr Ser Ala Ser Ala Ala Pro Ala Asp
            20                  25                  30


Lys Ala Thr Val Leu Ser Asn Trp Thr Gln Thr Ser Ala Ala Ser Tyr
        35                  40                  45


Asn Ala Phe Phe Ala Ala Arg Ala Asp Gln Gly Ala Trp Ser Ala Tyr

50                  55                  60

Gly Phe Asp Trp Ser Thr Asp Tyr Cys Thr Thr Ser Pro Asp Asn Pro
65                  70                  75                  80

Phe Gly Phe Pro Phe Ala Asn Ser Cys Val Arg His Asp Phe Gly Tyr
                85                  90                  95

Arg Asn Tyr Thr Ala Ala Gly Ser Phe Asp Ala Asn Lys Ser Arg Leu
            100                 105                 110

Asp Ser Ala Phe Tyr Ala Asp Leu Lys Arg Val Cys Thr Ala Tyr Ser
        115                 120                 125

Gly Val Lys Lys Ala Ser Cys Asp Ala Thr Ala Trp Thr Tyr Tyr Gln
    130                 135                 140

Ala Val Val Asn Leu Gly
145                 150

<210>  5
<211>  30
<212>  DNA
<213>  Artificial

<220>
<223>  Forward primer

<400>  5
ggaattccat atgcgccgtc gcgtcgccgc                                        30

<210>  6
<211>  30
<212>  DNA
<213>  Artificial

<220>
<223>  Reverse primer

<400>  6
cccaagcttt cagcccaggt tgacgaccgc                                        30

## Description

### Technical Field

The present invention relates to an enzyme composition for suppressing the occurrence of an unpleasant odor in foods and beverages, a method for suppressing the occurrence of an unpleasant odor, and a method for manufacturing a food with reduced unpleasant odor.

### Background Art

Phospholipase A2 (EC 3.1.1.4, hereinafter also referred to as "PLA2") is an enzyme that acts on the glycerophospholipid contained in soybean, egg yolk, rapeseed, sunflower, and the like, to hydrolyze the ester bond at position 2. PLA2 is widely found in microorganisms, animals, and plants. Examples of the PLA2 enzyme used industrially include PLA2 derived from porcine pancreas or actinomycetes, PLA2 obtained by expressing PLA2 enzyme derived from porcine pancreas in a mold using a genetic recombination technology, and the like (e.g., Patent Document 1).

It is known that the use of egg yolk or whole egg treated with PLA2 allows for the smooth release of bread and the like from a mould, the suppression of aging, and the texture of fluffiness and melt-in-the-mouth (Patent Document 2).

However, when egg yolk or whole egg, a fat or oil containing a phospholipid, or the like is acted on by an enzyme preparation containing a PLA2 as an active ingredient, and then used in foods such as bread and cake, there are some cases where an unpleasant odor occurs.

Thus, there is a demand for a method and an enzyme composition to prevent the occurrence of such an unpleasant odor and improve the flavor of foods.

### Prior Art Documents

### Patent Documents

Patent Document 1: Japanese Laid-Open Patent Publication No. 2011-172583
Patent Document 2: Japanese Laid-Open Patent Publication No. 2003-325140

### Summary of Invention

### Problems to be Solved by the Invention

An object of the present invention is to provide an enzyme composition capable of suppressing the occurrence of an unpleasant odor in foods and beverages, a method for suppressing the occurrence of an unpleasant odor in foods and beverages, and a method for manufacturing a food or beverage in which the occurrence of an unpleasant odor is suppressed.

### Means for Solving the Problems

The inventors of the present invention, by finding that a lipase may coexist in an enzyme preparation containing a PLA2 as an active ingredient, have considered that an unpleasant odor generated during the manufacture of bread or the like using modified (lyso-converted) egg yolk or modified (lyso-converted) whole egg obtained by treating egg yolk or whole egg with this enzyme preparation is mainly attributable to a free fatty acid and an oxide thereof resulting from fat and the like in the modified product due to the action of the lipase, and thus accomplished the present invention.

The present invention provides a flavor-improving enzyme composition for reducing an unpleasant odor in a food or beverage, the composition comprising an enzyme exhibiting phospholipase A2 activity with lipase activity/phospholipase A2 activity of not more than 0.005, as an active ingredient.

In one embodiment, the enzyme exhibiting phospholipase A2 activity is produced by a microorganism.

In one embodiment, the enzyme exhibiting phospholipase A2 activity is an enzyme derived from an actinomycete.

In one embodiment, the enzyme exhibiting phospholipase A2 activity is an enzyme encoded by a DNA composed of a base sequence represented by SEQ ID NO: 1 or 3 that may be with the substitution, deletion, insertion, and/or addition of one or more bases; or an enzyme composed of an amino acid sequence represented by SEQ ID NO: 2 or 4 that may be with the substitution, deletion, insertion, and/or addition of one to several amino acids.

The present invention provides a method for suppressing occurrence of an unpleasant odor in a food or beverage or a material of a food or beverage having a lyso-converted phospholipid, the method including:
treating a food or beverage or a material of a food or beverage that contains a phospholipid with the enzyme composition mentioned above.

In one embodiment, the material is egg yolk or whole egg.

In one embodiment, the material is a fat or oil containing a phospholipid.

In one embodiment, the food or beverage is pastry, bread, cake, mayonnaise, or dressing.

The present invention provides a method for manufacturing a food or beverage or a material of a food or beverage having a lyso-converted phospholipid, in which occurrence of an unpleasant odor is suppressed, the method including:
treating a food or beverage or a material of a food or beverage that contains a phospholipid with the flavor-improving enzyme composition mentioned above.

In one embodiment, the material is egg yolk or whole egg.

In one embodiment, the material is a fat or oil containing a phospholipid.

In one embodiment, the food or beverage is pastry, bread, cake, mayonnaise, or dressing.

The present invention also provides a food or beverage or a material of a food or beverage having a lyso-converted phospholipid, manufactured using the manufacturing method mentioned above.

### Effects of the Invention

According to the present invention, by finding that lipase activity coexists in an enzyme preparation exhibiting phospholipase A2 activity, it is possible to suppress the occurrence of an unpleasant odor in foods and beverages using an enzyme composition exhibiting phospholipase A2 activity with no or extremely low lipase activity. Moreover, according to the present invention, an enzyme composition is provided, which can be suitably used for the above-described purpose. By the use of such an enzyme composition together with a food or beverage or a material thereof, it is possible to provide a method for suppressing the occurrence of an unpleasant odor in a food or beverage and a method for manufacturing a food or beverage in which the occurrence of an unpleasant odor is suppressed. Furthermore, such a food or beverage in which the occurrence of an unpleasant odor is suppressed are provided.

### Mode for Carrying Out the Invention

Phospholipase A2 is an enzyme having a catalytic action of hydrolyzing the ester bond at position 2 of glycerophospholipids (e.g., phosphatidylcholine) to generate 1-acyl-2-lysophospholipid, which may be also referred to simply as "1-acyllysophospholipid" below and an example of which is 1-acyl-2-lysophosphatidylcholine, and a fatty acid.

An enzyme composition of the present invention is characterized in that an active ingredient of the enzyme composition is an enzyme exhibiting phospholipase A2 activity and having a ratio of lipase activity/phospholipase A2 activity of not more than 0.005. The ratio of lipase activity/phospholipase A2 activity is more preferably not more than 0.001 and even more preferably not more than 0.0001. Most preferably, the ratio of lipase activity/phospholipase A2 activity is 0, that is, the enzyme has no lipase activity.

In this specification, the phospholipase A2 activity and the lipase activity are measured using the following respective methods. The ratio of lipase activity/phospholipase A2 activity means the ratio of unit numbers as determined by the following respective methods.

With regard to the phospholipase A2 activity, when an enzyme is reacted with soybean lecithin serving as a substrate at 37°C and at pH 8.0, a degree of activity of which the enzyme releases 1 µmol of a free fatty acid in one minute is defined as 1 unit. The free fatty acid generated by the enzyme reaction is measured using a NEFA-C Test Wako (manufactured by Wako Pure Chemical Industries, Ltd.).

The lipase activity is measured by the following procedure using a Lipase Kit S (DS Pharma Biomedical Co., Ltd.). First, 50 µL of a sample is mixed with 1 mL of a chromogenic solution containing 5,5'-dithiobis(2-nitrobenzoic acid), and then 20 µL of an esterase inhibitor solution containing phenylmethylsulfonyl fluoride is added to and mixed with the mixture. After mixing, the resultant mixture is preheated in a thermostat bath at 30°C for 5 minutes. After the preheating is finished, 100 µL of a substrate solution containing dimercaprol tributyrate and sodium dodecyl sulfate is added to and mixed with the mixture, and after mixing, the resultant mixture is incubated at 30°C for 30 minutes. Then, the reaction is stopped by adding 2 mL of a stop solution to the mixture. The absorbance is measured at 412 nm. To a blank, the substrate solution is added after the reaction is stopped. The lipase activity is defined as 1 unit where indicating the difference in absorbance between the sample and the blank of 0.001.

The enzyme exhibiting phospholipase A2 activity is not limited with regard to the source of the enzyme and the method for preparing the enzyme as long as it satisfies the ratio of lipase activity/phospholipase A2 activity as mentioned above.

The source of the enzyme exhibiting phospholipase A2 activity may be, but is not particularly limited to, animals, plants, microorganisms, and the like. The enzyme may be isolated and purified from a microorganism, an animal, a plant, or the like , or may be an enzyme obtained by cloning a gene for the enzyme from a microorganism, an animal, a plant, or the like; producing a transformant by introducing the cloned gene into a microorganism or an animal or plant cell by genetic recombination; and extracting and purifying the enzyme produced by this transformant. Moreover, the enzyme may be an enzyme obtained by artificially modifying the above-described enzyme using a protein engineering approach or the like. The enzyme is preferably an enzyme derived from a microorganism and more preferably an enzyme derived from an actinomycete.

In one embodiment, the enzyme exhibiting phospholipase A2 activity is an enzyme produced by a microorganism. The origin of the enzyme produced by a microorganism may be not only an enzyme derived from the microorganism, but also an enzyme other than enzymes derived from microorganisms, including an enzyme derived from an animal, a plant, or the like but produced by the microorganism. As the enzyme derived from an animal, an enzyme derived from a swine is preferably used though the enzyme derived from an animal is not limited to this enzyme. Examples of the microorganism used for enzyme production includes bacteria, actinomycetes, yeast, filamentous fungi (molds), cyanobacteria, fungi, unicellular algae, and protozoans.

Examples of the microorganism that can be preferably used include, but is not limited to, naturally occurring wild-type microorganisms, naturally occurring and/or artificially induced mutants of the wild-type microorganisms, or transformants of these microorganisms. The enzyme is typically used as a purified or partially purified enzyme isolated or extracted from a microorganism cell or a culture solution of the cell. The purified enzyme may be used in a solution or may be used in a state of being immobilized on a column, beads, or the like. Alternatively, the microorganism cell may be used directly for the reaction.

Examples of the actinomycetes include actinomycetes belonging to the genus *Streptomyces*, the genus *Actinomadura,* or the genus *Frankia.*

The enzyme derived from a microorganism is preferably an enzyme derived from an actinomycete and more preferably an enzyme derived from an actinomycete belonging to the genus *Streptomyces*. As such an enzyme, for example, phospholipase A2 (trade name "PLA2 NAGASE 10P/R", manufactured by Nagase ChemteX Corporation) derived from the *Streptomyces violaceoruber* NBRC15146, phospholipase A2 derived from *Streptomyces avermitilis* NBRC14893, and the like are preferably used.

The phospholipase A2 derived from *S*. *violaceoruber* NBRC15146 satisfies the ratio as mentioned above, and preferably has the following physical and chemical properties:
(a) Action and substrate specificity: it hydrolyzes the linkage site of the acyl group at position 2 to the glycerol backbone in 1,2-diacylphospholipid to release a 2-acyl group.
(b) Optimum pH: 7 to 9
(c) Stable pH: 4 to 10
(d) Optimum temperature: 50°C
(e) Stable temperature: 30 to 60°C
(f) Molecular weight: 14 kDa as measured by SDS-PAGE

A naturally-occurring type of the phospholipase A2 derived from *S*. *violaceoruber* NBRC15146 has an amino acid sequence represented by SEQ ID NO: 2, and is encoded by a gene composed of a base sequence represented by SEQ ID NO: 1. The base sequence may also be a base sequence represented by SEQ ID NO: 1 with the substitution, deletion, insertion, and/or addition of one or more bases. The number of bases in the base sequence that are substituted, deleted, inserted, and/or added is more preferably 90 or less, even more preferably 45 or less, still more preferably 30 or less, yet more preferably 20 or less, still even more preferably 16 or less, yet even more preferably 5 or less, particularly preferably one to several, and most preferably 1 to 3.

Phospholipase A2 derived from *S*. *avermitilis* NBRC14893 satisfies the ratio as mentioned above, and can be prepared in the following manner, for example.

PCR amplification is performed using a genomic DNA prepared from *S*. *avermitilis* NBRC14893, as a template, with a forward primer of SEQ ID NO: 5 and a reverse primer of SEQ ID NO: 6 to obtain a gene fragment. pTONA5 (Japanese Laid-Open Patent Publication No. 2009-65837) is treated with restriction enzymes Nde I and HindIII, and the above gene fragment is treated with Nde I and HindIII. The obtained two fragments are ligated, and then introduced into *E. coli* JM109. A plasmid pTONA5 containing the gene fragment is obtained using kanamycin as a selection marker. The obtained plasmid containing the gene fragment is introduced into a conjugative *E. coli*, S17-1, and *Streptomyces lividans* 1326 is transformed by conjugative transformation using the transformed *E. coli.* This transformed strain is cultured to produce an enzyme. An enzyme solution is acquired from the culture solution, and the enzyme is separated and purified as necessary.

A naturally-occurring type of phospholipase A2 derived from *S*. *avermitilis* NBRC14893 has an amino acid sequence represented by SEQ ID NO: 4, and is encoded by a gene composed of a base sequence represented by SEQ ID NO: 3. With respect to the base sequence, the gene may also be composed of a base sequence represented by SEQ ID NO: 3 with the substitution, deletion, insertion, and/or addition of one or more bases. The number of bases in the base sequence that are substituted, deleted, inserted, and/or added is more preferably 90 or less, even more preferably 45 or less, still more preferably 30 or less, yet more preferably 20 or less, still even more preferably 16 or less, yet even more preferably 5 or less, particularly preferably one to several, and most preferably 1 to 3.

As long as the above-described ratio is satisfied, the enzyme, such as phospholipase A2 derived from *S*. *violaceoruber* NBRC15146 and phospholipase A2 derived from *S*. *avermitilis* NBRC14893, may also be an enzyme having an amino acid sequence of a native enzyme (amino acid sequence represented by SEQ ID NO: 2 or 4) with the substitution, deletion, insertion, and/or addition of one or more amino acids.

A person skilled in the art can alter the structure of proteins by, for example, appropriately introducing a substitution, deletion, insertion, and/or addition mutation using a site-specific mutation introduction method and the like usually used by those skilled in the art. In this case, it is preferable to introduce a mutation that has no adverse effect on the activity, taking the common technical knowledge such as homologous amino acids and active center into account.

In the present invention, the position, type, and number of amino acid residues that can be substituted, deleted, inserted, and/or added are not limited as long as the above-described ratio is satisfied. The number of amino acid residues may be, for example, 30 or less, more preferably 15 or less, even more preferably 7 or less, still even more preferably 5 or less, particularly preferably one to several, and most preferably 1 to 3. The position and type of amino acid residues are not limited as long as the above-described ratio is satisfied, for example.

Moreover, since mutations of amino acids may also occur in nature, not only enzymes in which amino acids have been artificially mutated but also enzymes in which amino acids have been mutated in nature can be used in the present invention as long as they satisfy the ratio as mentioned above.

A protein having an amino acid sequence having a certain level of identity (containing no substitution for an analogous amino acid) or homology (containing a substitution for an analogous amino acid) to the amino acid sequence of a native enzyme (e.g., amino acid sequence represented by SEQ ID NO: 2 or 4) may also be used in the present invention as long as the ratio as mentioned above is satisfied.

Preferably, the protein may be a protein having an amino acid sequence having at least 70%, more preferably at least 80%, even more preferably at least 90%, particularly preferably at least 95%, and still even more preferably at least 99% identity to the amino acid sequence represented by SEQ ID NO: 2 or 4.

The search for the identity or homology of proteins can be performed by, for example, searching a database of amino acid sequences of proteins, such as SWISS-PROT, PIR, or DAD; a database of DNA sequences, such as DDBJ, EMBL, or GenBank; a database of deduced amino acid sequences based on DNA sequences, or other databases using a homology search program such as BLAST, FASTA, ClustalW, or Phylip, through the Internet or using a local host, for example. The activity of proteins can be confirmed using the procedure as mentioned above.

The enzyme exhibiting phospholipase A2 activity may be encoded by a polynucleotide or a gene containing the base sequence represented by SEQ ID NO: 1 or 3, for example. The polynucleotide or the gene containing the base sequence represented by SEQ ID NO: 1 encodes a protein containing the amino acid sequence represented by SEQ ID NO: 2, that is, the phospholipase A2 derived from *S*. *violaceoruber* NBRC15146, and the polynucleotide or the gene containing the base sequence represented by SEQ ID NO: 3 encodes a protein containing the amino acid sequence represented by SEQ ID NO: 4, that is, the phospholipase A2 derived from *S*. *avermitilis* NBRC14893.

The enzyme that can be used in the present invention may also be an enzyme that is encoded by a polynucleotide that can be hybridized under stringent conditions with a polynucleotide having a base sequence complementary to the polynucleotide containing the base sequence represented by SEQ ID NO: 1 or 3, for example, and that satisfies the ratio as mentioned above.

The polynucleotide that can be hybridized under stringent conditions refers to a polynucleotide that is hybridized with a probe designed through the selection of one or more of the entire length of the phospholipase A2 protein-coding region in the base sequence represented by SEQ ID NO: 1 or 3, or any sequences of at least 20 and preferably at least 30, for example, 40, 60, or 100 contiguous bases in that coding region, using an ECL direct nucleic acid labeling and detection system (manufactured by Amersham Biosciences) under the conditions described in the manual thereof.

More specifically, "stringent conditions" usually refers to, but not particularly limited to, the conditions of, for example, 42°C, 2 × SSC, and 0.1% SDS, preferably 50°C, 2 × SSC, and 0.1% SDS, and further preferably 65°C, 0.1 × SSC, and 0.1% SDS. A plurality of factors, such as temperature, salt concentration, and formamide concentration, have an effect on the stringency of hybridization, and a person skilled in the art can realize optimal stringency by appropriately selecting these factors.

The present invention provides a flavor-improving enzyme composition for reducing an unpleasant odor in foods and beverages, the composition containing an enzyme exhibiting phospholipase A2 activity with lipase activity/phospholipase A2 activity of not more than 0.005 as an active ingredient. The "unpleasant odor" as used herein typically refers to, but is not limited to, unpleasant odors emanating from a fatty acid degraded by a lipase and an oxide of the fatty acid. Moreover, in the flavor-improving enzyme composition for reducing an unpleasant odor in foods and beverages according to the present invention, the lipase activity/phospholipase A2 activity may also be 0.

The flavor-improving enzyme composition may be composed of only the enzyme exhibiting phospholipase A2 activity with the lipase activity/phospholipase A2 activity of not more than 0.005, or may contain other ingredients, such as an excipient and an additive, that are acceptable in the field of foods as long as the ingredients substantially do not impair the enzyme activity. The amount of the enzyme exhibiting phospholipase A2 activity in the enzyme composition of the present invention is not particularly restricted as long as the enzyme treatment is achieved, but may be for example 0.05 to 100 % by weight, preferably 0.05 to 50 % by weight, and more preferably 0.05 to 30 % by weight. The enzyme composition can be used in any form. Although the form of the enzyme composition is not particularly limited as long as the enzyme treatment is achieved, for example, the enzyme composition may be used in liquid form in which the enzyme composition is dissolved or dispersed in a solvent such as water, or may be used in solid form (e.g., powder form).

The enzyme composition of the present invention is added to a food or beverage, or a material of a food or beverage, in which modification of a phospholipid is required, and is used for the enzyme treatment. Thus, the phospholipid can be modified, and an unpleasant odor that would be caused by treatment with other phospholipase A2 preparations is suppressed. Moreover, it is also possible that the enzyme composition of the present invention is mixed with a material containing no phospholipid, of materials of a food or beverage, then added to the other materials containing a phospholipid, and used for the enzyme treatment. Here, examples of the material containing no phospholipid include fats and oils (e.g., refined oil) containing no phospholipid. Especially for refined oil, since an unpleasant odor may occur due to lipase activity, it is difficult to be mixed with other phospholipase A2 preparations in advance. However, refined oil can be mixed with the enzyme composition of the present invention in advance. Moreover, in the cases where a fat or oil containing a phospholipid is used as a material, the enzyme treatment with the enzyme composition of the present invention makes it possible to obtain a fat or oil in which the phospholipid is lyso-converted while suppressing the occurrence of an unpleasant odor. The same holds for the method for suppressing the occurrence of an unpleasant odor according to the present invention, the manufacturing method of the present invention, and a food or beverage, or a raw material of a food or beverage, manufactured using the manufacturing method.

The present invention also provides a method (hereinafter, sometimes referred to as "the method of the present invention") for suppressing the occurrence of an unpleasant odor of a food or beverage or a material of a food or beverage having a lyso-converted phospholipid, the method including a step of treating a food or beverage or a material of a food or beverage with the enzyme composition. The form of the "enzyme composition" used for the treatment may be a liquid form or may be a solid form and can be appropriately selected depending on the food or beverage or the material of the food or beverage to be treated.

The target of the enzyme treatment with the flavor-improving enzyme composition of the present invention is a food or beverage or a material of a food or beverage, and examples of the target include egg yolk, whole egg, and fats and oils containing a phospholipid. In one embodiment, the target is egg yolk, whole egg, or a fat or oil containing a phospholipid. The egg yolk, which is to be treated with the enzyme composition of the present invention, can be used in any form such as raw, frozen, powdered, salted, or sugared form. The material of a food or beverage to be treated with the enzyme composition may also be in the form of whole egg, which includes egg white. Moreover, fats and oils that can be used as a material of bread, cake, and the like may be modified with the enzyme composition. Moreover, examples of a fat or oil containing a phospholipid include margarine, cream, mayonnaise, dressing, chocolate, shortening, and fat spread.

In one embodiment, a food or beverage made with egg yolk, whole egg, or a fat or oil modified using the flavor-improving enzyme composition of the present invention is provided. Examples of such food or beverage include pastry, bread, cake, mayonnaise, and dressing.

The "food or beverage" or the "material of a food or beverage" also includes edible fats and oils. In one embodiment, the "food or beverage" or the "material of a food or beverage" is a modified fat or oil with reduced unpleasant odor, obtained by treating a fat or oil with the flavor-improving enzyme composition.

Although the term "pastry" as used herein is not particularly limited as long as it is pastry in which flour or other grains are used, the "pastry" refers to Manju (steamed bread), Mushi-yokan (steamed adzuki-bean jelly), Castilla (sponge cake), Dorayaki (two small pancakes with bean jam in between), Imagawa-yaki (Japanede muffin containing bean jam, served hot), Taiyaki (fish-shaped pancake filled with bean jam), Kintsuba (confection of sweetened beans wrapped in wheat-flour dough), waffle, Kurimanju (chestnut bread), Geppei (mooncake), Bolo (small round cookie), Yatsuhashi (cinnamon-seasoned cracknel), rice cracker, Karintou (fried dough cookie), cookie, doughnut, or the like.

The term "cake" as used herein refers to sponge cake, butter cake, chiffon cake, roll cake, Swiss roll, bouchée (biscuit), Baumkuchen, pound cake, cheesecake, snack cake, steamed cake, hotcake, pancake or the like.

The term "fat or oil" as used herein means an edible fat or oil, and examples thereof include, but not limited to, those (fatty oil) that are liquid at normal temperature and those (fat) that are solid at normal temperature, as mentioned below. Examples of the fatty oil (liquid at normal temperature) include salad oil, essential rape oil, corn oil, soybean oil, sesame oil, rapeseed oil (canola oil), rice oil, rice bran oil, camellia oil, safflower oil (*Carthamus tinctorius* oil), coconut oil (palm kernel oil), cottonseed oil, sunflower oil, perilla oil (PERILLA OIL), linseed oil, olive oil, peanut oil, almond oil, avocado oil, hazelnut oil, walnut oil, grapeseed oil, mustard oil, lettuce oil, whale oil, shark oil, and cod-liver oil. Examples of the fat (solid at normal temperature) include cocoa butter, peanut butter, palm oil, lard (pork fat), tallow (beef tallow), chicken fat, rabbit fat, mutton tallow, horse fat, schmaltz, milkfat (butter, ghee, and the like), and hydrogenated oil (margarine, shortening, and the like).

Moreover, a material, such as egg yolk, whole egg, or a fat or oil containing a phospholipid, of a food or beverage, that is treated with the enzyme composition of the present invention, may also be preferably used in acidic oil-in-water emulsions such as mayonnaise, tartare sauce, and emulsified dressing.

The step of treating a food or beverage or a material of a food or beverage with water and the enzyme composition is regardless of the order or manner in which the enzyme is added, as long as the enzyme composition added to a food or beverage or a material of a food or beverage can act on the phospholipid in the food or beverage or the material of the food or beverage. The step of treating a food or beverage or a material of a food or beverage with the enzyme is generally performed in a pretreatment step of the material in manufacturing foods and beverages as usually practiced. For the step of treating a food or beverage or a material of a food or beverage with the enzyme composition, the enzyme can be added, for example, in a form of dispersing or dissolving the enzyme in water or an appropriate aqueous solution.

In the present invention, the enzyme treatment refers to a treatment in which a food or beverage or a material of a food or beverage is brought into contact with the enzyme composition of the present invention (which may be mixed with other raw materials of the food or beverage) so that phospholipase A2 in the enzyme composition can act on the substrate.

The enzyme treatment is performed within a pH range of preferably 2 to 10, more preferably 3 to 9, and even more preferably 4 to 9. In order to achieve the above-described pH, an alkali (e.g., sodium hydroxide) may be added before, at the same time as, or after the addition of the enzyme.

The enzyme treatment may be performed under the conditions of usually 25 to 80°C and preferably 30 to 60°C usually for 15 minutes to 8 hours and preferably for 15 minutes to 5 hours, depending on the optimal temperature of the enzyme.

The amount of the enzyme composition that is used for the enzyme treatment is not particularly limited as long as the modification (lyso-convertion) of the target to be treated can be achieved, but is such an amount that allows the phospholipase A2 activity to be preferably 100 to 5000 units and more preferably 300 to 3000 units with respect to 1 kg of a food or beverage or a material of a food or beverage. Moreover, the amount of the enzyme composition that is used for the enzyme treatment may also be preferably 1 to 1000 ppm and more preferably 10 to 500 ppm per enzyme treatment. The enzyme treatment may be performed in a batch manner, a multistage batch manner, or a continuous manner. Desirably, heat treatment is performed after the enzyme treatment in order to inactivate the enzyme.

The present invention also provides a method (hereinafter sometimes referred to as the "manufacturing method of the present invention") for manufacturing a food or beverage in which the occurrence of an unpleasant odor is suppressed, and this manufacturing method includes the steps of treating a material of the food or beverage with the above-described enzyme composition; and manufacturing the food or beverage using the material treated with the enzyme composition. The treatment of a material of the food or beverage with the enzyme composition is as described above. To manufacture a food or beverage using the treated material, a common procedure used in the art may be adopted.

The present invention also provides a food or beverage manufactured using the above-described manufacturing method. Such food or beverage may be a food or beverage in which the occurrence of an unpleasant odor, which may be caused by treatment with other phospholipase A2 enzyme preparations, is suppressed.

### Examples

Hereinafter, the present invention will be more specifically described by means of examples, however, the present invention is not limited by these examples.

### Example 1: Ratio of lipase activity/phospholipase A2 activity

The phospholipase A2 activity and the lipase activity were measured with respect to PLA2 derived from an actinomycete (PLA2 NAGASE 10P/R) manufactured by Nagase ChemteX Corporation; PLA2 derived from porcine pancreas (Biocatalysts Ltd., product name: Lipomod 699L), and PLA2 obtained by expressing PLA2 derived from porcine pancreas in a mold (DSM, product name: Maxapal A2).

### Assay for measuring PLA2 activity

With respect to the phospholipase A2 activity, when the enzyme was reacted with soybean lecithin serving as the substrate at 37°C and at pH 8.0, a degree of activity of which the enzyme released 1 µmol of a free fatty acid in one minute was defined as 1 unit. The free fatty acid generated by the enzyme reaction was measured using NEFA-C Test Wako (Wako Pure Chemical Industries, Ltd.).

### Assay for measuring lipase activity

The lipase activity was measured using a Lipase Kit S (DS Pharma Biomedical Co., Ltd.) according to the following procedure. First, 50 µL of a sample was mixed with 1 mL of a chromogenic solution containing 5,5'-dithiobis(2-nitrobenzoic acid), and then 20 µL of an esterase inhibitor solution containing phenylmethylsulfonyl fluoride was added to and mixed with the mixture. After mixing, the resultant mixture was preheated in a thermostat bath at 30°C for 5 minutes. After the preheating was finished, 100 µL of a substrate solution containing dimercaprol tributyrate and sodium dodecyl sulfate was added to and mixed with the mixture, and then, the resultant mixture was incubated at 30°C for 30 minutes. Then, the reaction was stopped by adding 2 mL of a stop solution to the mixture. The absorbance was measured at 412 nm. To a blank, the substrate solution was added after the reaction was stopped. The lipase activity was defined as 1 unit where indicating the difference in absorbance between the sample and the blank of 0.001.

### Comparison of phospholipase A2 activity with lipase activity

As shown in Table 1, it was found that the PLA2 derived from an actinomycete or the PLA2 derived from porcine pancreas had a ratio of the lipase activity in the phospholipase A2 activity significantly lower than that of the PLA2 obtained by expressing the PLA2 derived from porcine pancreas in a mold.

**[Table 1]**

| | PLA2 NAGASE 10P/R | Lipomod 699L | Maxapal A2 |
|---|---|---|---|
| Phospholipase A2 Activity | 100,000 U/g | 26,000 U/mL | 30,000 U/mL |
| Lipase Activity | N. D. | N. D. | 170 U/ mL |
| Lipase Activity/ Phospholipase A2 Activity | 0 | 0 | 0.0057 |

### Example 2: Evaluation of manufactured bread

Materials shown in Table 2 were put into a mixer (Aicohsha Manufacturing Co., Ltd.), other than butter, and kneaded at 26°C at a low speed for 3 minutes, at a low-medium speed for 3 minutes, and at a low-medium speed for 5 minutes. Then, unsalted butter was put into the mixer, and kneading was performed at a low speed for 2 minutes, at a medium-low speed for 5 minutes, at a high speed for 1 minute, and at a medium-low speed for 2 minutes, followed by primary fermentation (at 30°C in a humidity of 75% for 90 minutes). After the end of the primary fermentation, the dough was divided into fractions of 237.5 g each, degassing was performed using a moulder (manufactured by Aicohsha Manufacturing Co., Ltd.), and then shaping was performed. Then, secondary fermentation was performed (at 32°C in a humidity of 75% for 70 minutes), and baking was performed in an oven at 230°C for 40 minutes. A group in which no enzyme was added was set as a control group. With respect to the used amounts of the enzymes, the enzymes were added in such amounts of the enzymes to be equated for the unit numbers (U) of the activity.

**[Table 2]**

| Materials | PLA2 NAGASE 10P/R | Lipomod 699L | MaxapalA2 | Control |
|---|---|---|---|---|
| Hard Flour | 525g | 525g | 525g | 525g |
| Soft Flour | 225g | 225g | 225g | 225g |
| Sugar | 75g | 75g | 75g | 75g |
| Salt | 15g | 15g | 15g | 15g |
| Fat-free Milk | 15g | 15g | 15g | 15g |
| Unsalted Butter | 187. 5g | 187. 5g | 187. 5g | 187.5g |
| Bakery Yeast | 22. 5g | 22. 5g | 22. 5g | 22. 5g |
| Whole Egg | 187.5g | 187.5g | 187.5g | 187.5g |
| Water | 300g | 300g | 300g | 300g |
| PLA2 NAGASE 10P/R | 0.52g (57200U) | - | - | - |
| Lipomod 699L | - | 2.16g (56250U) | - | - |
| MaxapalA2 | - | - | 1. 875g (56250U) | - |

The manufactured bread was evaluated by three evaluators with respect to an unpleasant odor. The evaluation was performed one day after baking and three days after baking. The evaluation was performed on the basis of three grades: grade of 1, no unpleasant odor; grade of 2, having an unpleasant odor; and grade of 3, having an especially strong unpleasant odor. Table 3 shows the results.

**[Table 3]**

| Days after Baking | Example 1 (PLA2 NAGASE 10P/R) | Example 2 (Lipomod 699L) | Comparative Example 1 (Maxapal A2) | Control (No Enzyme) |
|---|---|---|---|---|
| D+1 | 1 | 1 | 3 | 1 |
| D+3 | 1 | 1 | 3 | 1 |

As shown in Table 3, it was confirmed that the PLA2 obtained by expressing PLA2 derived from porcine pancreas in a mold caused the occurrence of an unpleasant odor in the bread after baking, compared with the PLA2 derived from an actinomycete or the PLA2 derived from porcine pancreas, in an equivalent activity.

### Example 3: Measurement of the amount of free fatty acid in enzyme-treated bread

Materials shown in Table 4 were put into a mixer (Aicohsha Manufacturing Co., Ltd.), other than butter, and kneaded at 26°C at a low speed for 3 minutes and at a low-medium speed for 2 minutes. Then, butter was put into the mixer, and kneading was performed at a low-medium speed for 2 minutes and at a medium-low speed for 7 minutes, followed by primary fermentation (at 30°C in a humidity of 90% for 30 minutes). After the end of the primary fermentation, the dough was divided into fractions of 220 g each, degassing was performed using a moulder (Aicohsha Manufacturing Co., Ltd.), and then shaping was performed. Then, secondary fermentation was performed, and baking was performed in an oven at 210°C for 40 minutes. A group in which no enzyme was added was set as a control group.

**[Table 4]**

| | PLA2 NAGASE 10P/R | Lipomod 699L | Maxapal A2 | Control |
|---|---|---|---|---|
| Hard Flour | 800g | 800g | 800g | 800g |
| Sugar | 48g | 48g | 48g | 48g |
| Salt | 16g | 16g | 16g | 16g |
| Fat-free Milk | 8g | 8g | 8g | 8g |
| Unsalted Butter | 32g | 32g | 32g | 32g |
| Bakery Yeast | 24g | 24g | 24g | 24g |
| Egg York | 80g | 80g | 80g | 80g |
| Ascorbic Acid | 4g | 4g | 4g | 4g |
| Water | 556g | 556g | 556g | 556g |
| PLA2 NAGASE 10P/R | 0.131g 15589U | - | - | - |
| Lipomod 699L | - | 0.6g 15600U/g | - | - |
| Maxapal A2 | - | - | 0.52g 15600U | - |

The amount of free fatty acid in the enzyme-treated bread was measured by GC-headspace analysis using (GC2010 manufactured by Shimadzu Corporation) for GC. About 2.1 g to about 2.3 g of the crumb on the next day after baking was collected into a sample vial (manufactured by GL Sciences Inc.) for headspace and analyzed as a sample.

### <GC>

Column: InertCap
FFAP (0.25 mm ID × 30 m, df = 0.25 µm) (manufactured by GL Sciences Inc.)
Column temperature: 60°C-10°C/min-240°C (5 min)
Detector: FID, 240°C
Carrier gas: He
Column flow rate: 1.2 mL/min

### <Headspace>

Incubation time: 30 min
Oven temperature: 90°C
Transfer temperature: 210°C
Needle temperature: 200°C
Carrier gas pressure: 140 kPa

The peak areas of acetic acid (C2), propionic acid (C3), butyric acid (C4), isobutyric acid (Ci4), valeric acid (C5), isovaleric acid (Ci5), capronic acid (C6), enanthic acid (C7), caprylic acid (C8), and capric acid (C10) were calculated. The sum of the peak areas was divided by the weight of the bread analyzed. The amount of fatty acid generated by each enzyme preparation was calculated as compared to 100 for the group in which no enzyme was added.

As shown in Table 5, it was found that the PLA2 obtained by expressing PLA2 derived from porcine pancreas in a mold (Maxapal A2) produces a significantly larger amount of fatty acid than the other PLA2s. These results agree with the data on the lipase content in Example 1, and thus there is a possibility of involvement of the lipase in Maxapal A2.

**[Table 5]**

| | Example 3 (PLA2 NAGASE 10P/R) | Example 4 (Lipomod 699L) | Comparative Example 2 (Maxapal A2) | Control (No Enzyme) |
|---|---|---|---|---|
| Amount of Free Fatty Acid | 96 | 89 | 118 | 100 |

### Industrial Applicability

According to the present invention, it is possible to manufacture foods and beverages with reduced unpleasant odor while having a favorable flavor. Thus, the present invention can be used in the food industry and the food additives industry.

## Claims

1. A flavor-improving enzyme composition for reducing an unpleasant odor in a food or beverage, the composition comprising an enzyme exhibiting phospholipase A2 activity with lipase activity/phospholipase A2 activity of not more than 0.005, as an active ingredient.

2. The flavor-improving enzyme composition of claim 1, wherein the enzyme exhibiting phospholipase A2 activity is produced by a microorganism.

3. The flavor-improving enzyme composition of claim 1 or 2, wherein the enzyme exhibiting phospholipase A2 activity is an enzyme derived from an actinomycete.

4. A method for suppressing occurrence of an unpleasant odor in a food or beverage or a material of a food or beverage having a lyso-converted phospholipid, the method comprising:
treating a food or beverage or a material of a food or beverage that contains a phospholipid with the flavor-improving enzyme composition of any one of claims 1 to 3.

5. The method of claim 4, wherein the material is egg yolk or whole egg.

6. The method of claim 4 or 5, wherein the food or beverage is pastry, bread, cake, mayonnaise, or dressing.

7. A method for manufacturing a food or beverage or a material of a food or beverage having a lyso-converted phospholipid, in which occurrence of an unpleasant odor is suppressed, the method comprising:
treating a food or beverage or a material of a food or beverage that contains a phospholipid with the flavor-improving enzyme composition of any one of claims 1 to 3.

8. The manufacturing method of claim 7, wherein the material is egg yolk or whole egg.

9. The manufacturing method of claim 7 or 8, wherein the food or beverage is pastry, bread, cake, mayonnaise, or dressing.

10. A food or beverage or a material of a food or beverage having a lyso-converted phospholipid, manufactured using the manufacturing method of any one of claims 7 to 9.
